(19) Europäisches Patentamt
European Patent Office
Office européen des brevets

(11) EP 4 671 237 A1

(12) **EUROPEAN PATENT APPLICATION**

(43) Date of publication:
**31.12.2025 Bulletin 2026/01**

(21) Application number: **24184448.9**

(22) Date of filing: **25.06.2024**

(51) International Patent Classification (IPC):
***C07D 265/10*** *(2006.01)*

(52) Cooperative Patent Classification (CPC):
**C07D 265/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Studiengesellschaft Kohle gGmbH**
**45470 Mülheim (DE)**

(72) Inventors:
- **LIST, Benjamin**
  **45470 Mülheim an der Ruhr (DE)**
- **GUILLEN MORALEJO, Maria de los Angeles**
  **45468 Mülheim an der Ruhr (DE)**
- **DIAZ-OVIEDO, Christian David**
  **42275 Wuppertal (DE)**

(54) **PROCESS FOR THE ASYMMETRIC CYCLOADDITION OF ALKENES TOWARD 1,3-AMINO ALCOHOLS**

(57) The present invention relates to a process for the asymmetric cycloaddition of an iminium ion precursor to alkenes whereby 1,3-amino alcohols are obtained.

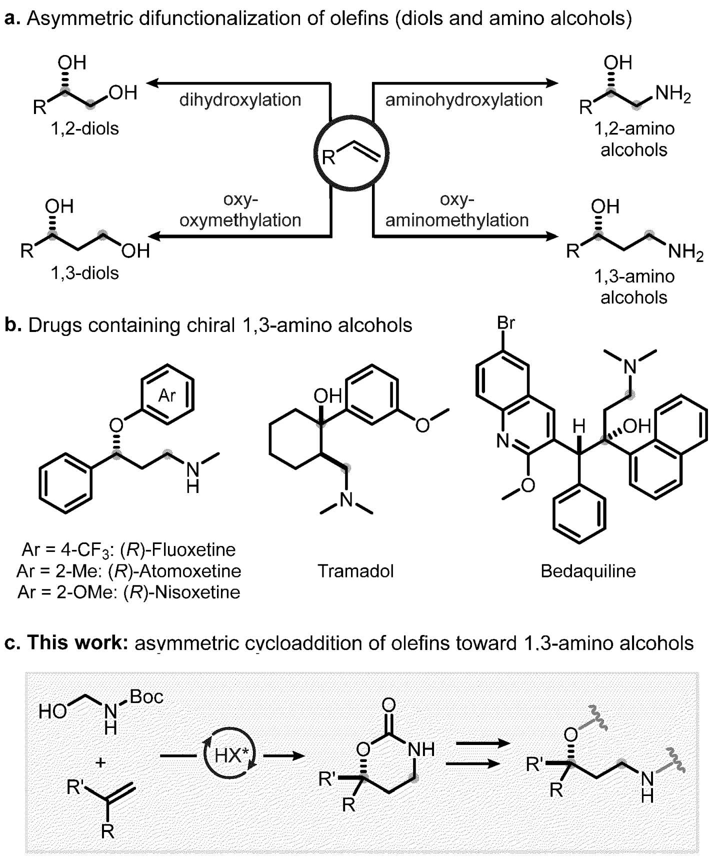


Figure 1



EP 4 671 237 A1

## Description

The present invention relates to a process for the asymmetric cycloaddition of an iminium ion precursor to alkenes whereby 1,3-amino alcohols are obtained.

Enantioenriched 1,3-amino alcohols are ubiquitous structural motives in natural products and active pharmaceutical ingredients. In the present application, the enantioselective strong Brønsted acid-catalyzed [4+2] cycloaddition of unbiased olefins and iminium ions is disclosed, which provides a one-step approach for the direct transformation of unbiased alkenes into highly functionalized molecules under mild reaction conditions.

As cost-effective and abundant materials, olefins have become pivotal in chemical synthesis. They are readily available in bulk quantities from renewable resources and petrochemical feedstocks, rendering them one of the most versatile classes of organic compounds capable of undergoing a plethora of transformations.

Their direct functionalization toward the rapid formation of structurally complex building blocks is amenable and, over the past century, led to transformations that include dihydroxylation and aminohydroxylation reactions, which allow respectively for the direct synthesis of valuable 1,2-diols and 1,2-amino alcohols (Figure 1A). The ubiquity of oxygen and nitrogen-based scaffolds in biologically active compounds continues to drive the advancement of existing methodologies and the development of new chemical tools. Additionally, the transformation of olefins into 1,3-dioxygenated moieties through their reaction with aldehydes (Prins reaction) has been extensively documented and several catalytic methodologies thereof are available nowadays. Nonetheless, an analogous, straightforward strategy that enables the oxy-aminomethylation of alkenes remains elusive, with only a few reports of non-asymmetric variants in the literature to date.

Enantioenriched 1,3-amino alcohols are consolidated building blocks in the pharmaceutical industry, especially for the synthesis of marketed blockbuster antidepressants drugs such as (*S*)-Duloxetine, (*R*)-Fluoxetine, and (*R*)-Atomoxetine (Figure 1B). In this context, enantiomerically enriched 1,3-amino alcohols have been synthesized from aldehydes or ketones via asymmetric hydrogenation with transition metal catalysts, as well as via hydroamination of allylic alcohols, intermolecular C-H amination of prefunctionalized substrates, and aza-aldol reactions. Nevertheless, reports on the synthesis of 1,3-amino alcohols through alkene functionalization remain notably underexplored. Inspired by the work of Hossain (Tetrahedron Letters 2011, 52 (33), 4353-4356) on the catalytic tandem elimination-cycloaddition of Boc-imines, the inventors considered that a single-step transformation of unbiased olefins to chiral oxazinanones, which are often found as key structural elements in bioactive molecules or serve as chiral precursors in drug synthesis, might address the above-mentioned challenges.

On the basis of the continuous efforts of the inventors in exploring chemical transformations of unfunctionalized early stage chemical feedstocks, the inventors have developed their work on the Bronsted acid-catalyzed [4+2] cycloaddition of alkenes and iminium ions to address precursors of industrially relevant and biologically active product motifs (Figure 1C).

In more detail, the present invention is directed to a process for the asymmetric cycloaddition of an iminium ion precursor to an alkene as represented in the following reaction scheme:

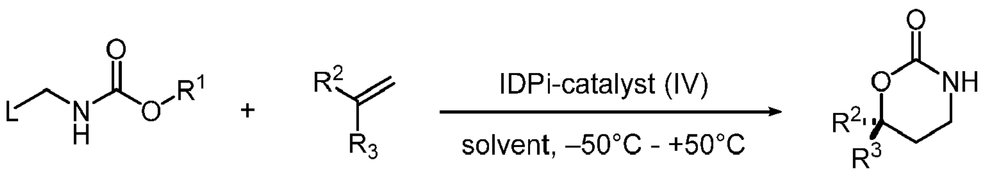


(I) (II) (III)

wherein an iminium ion precursor (I) is reacted with an alkene of Formula (II) in the presence of a catalyst of the Formula (IV), preferably in the presence of an organic solvent, whereby a 1 ,3-oxazinan-2-one of Formula (III) is obtained,
wherein:

$R^1$ represents H, $C_1$-$C_{18}$-alkyl, including tert-butyl, 3-ethylpentan-3-yl, 3-methylpentan-3-yl, tert-pentyl, $C_6$-$C_{14}$ aryl or $C_1$ -$C_6$ alkyl substituted $C_6$-$C_{14}$ aryl including benzyl, 2-phenylpropan-2-yl, 1,1-diphenylethyl,
L stands for a leaving group selected from hydroxy, alkoxy, acetate, sulfonate and halogen, $R^2$ represents H, $C_1$-$C_6$-alkyl, $C_1$-$C_6$-cycloalkyl, $C_1$-$C_8$-alkenyl, $C_1$-$C_8$-alkynyl, $C_6$ to $C_{18}$ aromatic hydrocarbon or $C_5$ to $C_{18}$ heteroaromatic hydrocarbon, each aromatic or heteroaromatic hydrocarbon optionally being substituted by one or more substituents selected from halogen, $C_1$ to $C_{20}$ straight chain, branched chain or cyclic aliphatic hydrocarbons, $C_2$ to $C_6$ alkenyl, $C_6$ to $C_{18}$ aromatic hydrocarbons or $C_5$ to $C_{18}$ heteroaromatic hydrocarbons, optionally having one or more halogens, preferably F and/or Cl, $SF_5$, $NO_2$ or cyano on the aliphatic, aromatic or heteroaromatic hydrocarbon,

$R^3$ represents hydrogen, $C_1$-$C_6$-alkyl, or -$C_1$-$C_6$-cycloalkyl, each alkyl optionally being substituted by halogen, and

wherein the IDPI-catalyst is represented by Formula (IV):

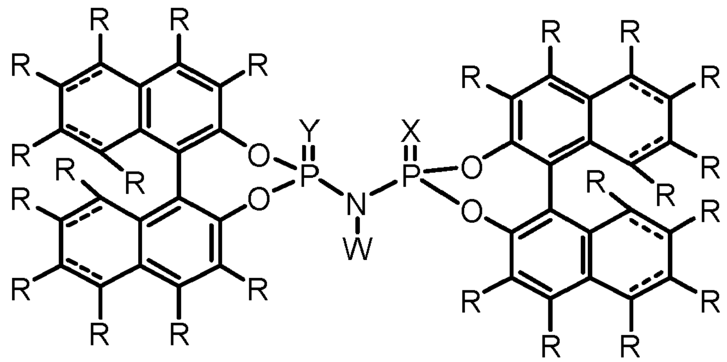

(IV)

wherein in said formula (IV):

R is the same or different on each position and is each selected from hydrogen, halogen, $SF_5$, $NO_2$, cyano, $C_1$ to $C_{20}$ straight chain, branched chain or cyclic aliphatic hydrocarbons,
optionally having one or more halogens, preferably F or Cl, $SF_5$, $NO_2$ or cyano on the aliphatic hydrocarbon, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or
$C_5$ to $C_{18}$ heteroaromatic hydrocarbons optionally having one or more halogens, preferably F and/or Cl, $SF_5$, $NO_2$ or cyano on the aliphatic or aromatic hydrocarbon,
wherein any dashed line independently represents a double bond or two hydrogens,
wherein X and Y are the same or different and represent $NR^N$; wherein:

$R^N$ is an electron withdrawing group, being the same or different on each position and being selected from:

i. -alkyl, -CO-alkyl, -(CO)-O-alkyl, sulfinyl alkyl, sulfonyl alkyl, sulfonyl iminoalkyl, sulfonyl bisiminoalkyl, phosphinyl dialkyl, phosphonyl alkyl, alkyl phosphorane, N,N'-alkylimidazolidin-2-iminyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or Cl, cyano, nitro, or $SF_5$;
ii. -aryl, -CO-aryl, -(CO)-O-aryl, sulfinyl aryl, sulfonyl aryl, sulfonyl iminoaryl, sulfonyl iminosulfonylaryl, sulfonyl bisiminoaryl, phosphinyl diaryl, phosphinyl alkylaryl, phosphonyl aryl, aryl phosphoranes, aryl alkyl phosphoranes, N,N'-arylimidazolidin-2-iminyl, N-aryl-N'-alkylimidazolidin-2-iminyl, wherein aryl is a $C_6$ to $C_{18}$ aromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;
iii. -heteroaryl, -CO-heteroaryl, -(CO)-O-heteroaryl, sulfinyl heteroaryl, sulfonyl heteroaryl, -(P=O)-di-heteroaryl, phosphinyl diheteroaryl, phosphinyl aryl heteroaryl, phosphinyl heteroaryl alkyl, phosphonyl heteroaryl, heteroaryl phosphoranes, heteroaryl aryl phosphoranes, heteroaryl aryl alkyl phosphoranes, N,N'-heteroarylimidazolidin-2-iminyl, N-heteroaryl-N'-alkylimidazolidin-2-iminyl, N-heteroaryl-N'-arylimidazolidin-2-iminyl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$; and

wherein W is selected from hydrogen, halogen, a metal selected from Li, Na, K, Rb, Cs, Be, Mg, Ca, Sr, Ba Sc, Ti, V, Cr, Mn, Fe, Co, Ni, Cu, Zn, Y, Zr, Mo, Ru, Rh, Pd, Ag, Cd, W, Re, Os, Ir, Pt, Au, Hg, Al, Ga, In, Ge, Sn, Pb, As, Sb, Bi, Se, Te, La, Sm, Eu, Yb, U or a cationic organic group, a substituted borane $-BR^IR^{II}R^{III}$ or a substituted silicon $-SiR^IR^{II}R^{III}$, wherein

$R^1$, $R^{II}$ and $R^{III}$ may be same or different and each stands for hydrogen, halogen, an optionally -O-bonded $C_1$ to $C_{20}$ straight chain, branched chain or cyclic aliphatic hydrocarbon, optionally having one or more unsaturated bonds or one or more hetero atoms in the chain, a $C_5$ to $C_{18}$ heteroaromatic hydrocarbon, a $C_6$ to $C_{18}$ aromatic hydrocarbon or partially arene-hydrogenated forms thereof, each hydrocarbon optionally being substituted by one or more groups selected from $C_1$ to $C_{20}$ straight chain, branched chain or cyclic aliphatic hydrocarbons, or one or more heterosubstituents, W being preferably selected from hydrogen, alkali metal or earth alkaline metal.

In an embodiment of the inventive process for Formula (I), (II) and (III),

PG, L and the catalyst of Formula (IV) are as defined above,
$R^1$ represents H, $C_1$-$C_6$-alkyl, or -$C_1$-$C_6$-cycloalkyl, $C_6$ to $C_{18}$ aromatic hydrocarbon or $C_5$ to $C_{18}$ heteroaromatic hydrocarbon, each aromatic or heteroaromatic hydrocarbon optionally being substituted by one or more substituents selected from halogen, $C_1$ to $C_{20}$ straight chain, branched chain or cyclic aliphatic hydrocarbons, $C_2$ to $C_6$ alkenyl, $C_6$ to $C_{18}$ aromatic hydrocarbons or $C_5$ to $C_{18}$ heteroaromatic hydrocarbons optionally having one or more halogens, preferably F and/or Cl, $SF_5$, $NO_2$ or cyano on the aliphatic or aromatic hydrocarbon,
$R^2$ represents hydrogen, $C_1$-$C_6$-alkyl, or -$C_1$-$C_6$-cycloalkyl, each alkyl optionally being substituted by halogen.

In another embodiment of the inventive process, the catalyst is represented by the following formula (IVa) or (IVb) :

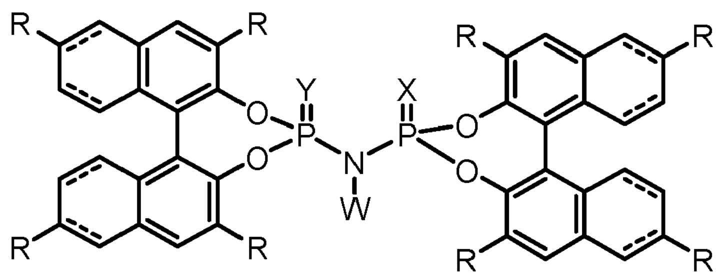

(IVa)

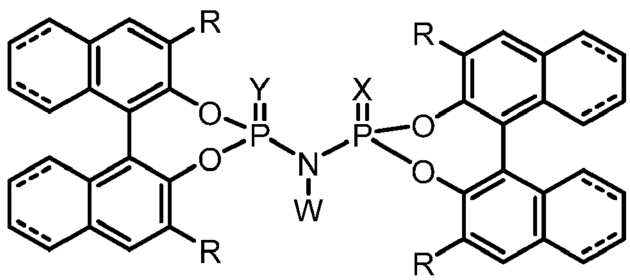

(IVb)

wherein the substituent R is the same or different on each position and is defined as in claim 1,
X and Y have the meanings as defined in claim 1,
any dashed line independently represents two hydrogens or preferably a double bond and W represents hydrogen, an alkali metal or an earth alkaline metal.

In another embodiment of the inventive process, the substituent R is the same or different on each position and represents halogen, a straight chain, branched chain or cyclic $C_1$ to $C_{20}$ aliphatic hydrocarbon or a $C_6$ to $C_{18}$ aromatic hydrocarbon, said aliphatic hydrocarbon and/or aromatic hydrocarbon being substituted by one or more halogens, preferably F and/or Cl, $SF_5$, $NO_2$ or a straight chain, branched chain or cyclic $C_1$ to $C_{20}$ aliphatic hydrocarbon, optionally being substituted by one or more halogens, preferably F and/or Cl, $SF_5$, $NO_2$ on the aliphatic hydrocarbon.

In a further embodiment of the inventive process as defined above for any formula (IV), (IVa) or (IVb), X and Y are the same or different and represent $NR^N$, wherein $R^N$ is an electron withdrawing group, being the same or different on each position and being selected from:

i. sulfinyl alkyl or sulfonyl alkyl, wherein alkyl is a $C_1$ to $C_{20}$ straight chain, branched chain or cyclic aliphatic

hydrocarbon, optionally having at least one substituent selected from $C_1$ to $C_6$ alkoxy, halogen, preferably F and/or C!, cyano, nitro or $SF_5$;

ii. sulfinyl aryl, or sulfonyl aryl, wherein aryl is a $C_6$ to $C_{18}$ aromatic hydrocarbon, optionally having at least one substituent selected from $C_1$ to $C_6$ alkyl optionally substituted by at least one halogen , $C_1$ to $C_6$ alkoxy, halogen, preferably F and/or C!, cyano, nitro or $SF_5$;

iii. sulfinyl heteroaryl, or sulfonyl heteroaryl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or C!, cyano, nitro or $SF_5$; and

wherein R and W have the meanings as defined in any one of the preceding claims.

In yet another embodiment of the inventive process , the catalyst is represented by Formula (IVc)

(IVc)

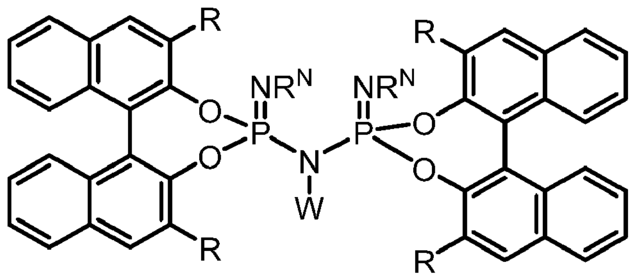

wherein R is the same on each position and is selected from hydrogen, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or $C_5$ to $C_{18}$ heteroaromatic hydrocarbons, optionally having one or more halogens, preferably F and/or Cl, $SF_5$, $NO_2$ or cyano on the aliphatic, aromatic or heteroaromatic hydrocarbon.

The reaction conditions for the inventive process are not critical and the reaction can be carried out in a temperature range from -100°C to 30°C or even higher up to 80°C, preferably in the range of -50°C to +50°C. The reaction can be carried out neat, in an aprotic organic solvent such as $CH_2Cl_2$, $CHCl_3$, $Et_2O$, THF, PhMe, pentane, hexane, cyclohexane, or in a protic organic solvent such as methanol, ethanol, generally under atmospheric pressure. Generally, the alkene is used in an excess of 2 to 20 mol equivalents to the iminium ion precursor, depending on the specific reaction partners and conditions.

The catalyst as represented by formulae (IV), (IVa), (IVb) and (IVc) may be used as such in in the reaction system or in an immobilized form where the catalyst is bonded to a carrier or forms part thereof, prepared according to the process as described in EP23198382.6.

In the formulae (IV), (IVa), (IVb) and (IVc) , the broken/dashed line might optionally present a double bond, thus demonstrating a naphthalene ring system, or a hydrogenated double bond, demonstrating a 4H-naphthalene ring system, and both forms might be present in the catalysts as used in the inventive process.

The catalyst used here is based on imidodiphosphate (IDP) catalyst, the iminoimidodiphosphorimidate (iIDP) catalyst (List et al., J. Am. Chem. Soc. 2016, 138, 34, 10822) and imidodiphosphorimidate (IDPi) catalyst and may be prepared using the process as described in EP20200632.6. Used solvents are dried before use.

Definitions

The following definitions apply to the individual groups R, $R^N$ and W equally as follows.

A heterosubstituent as defined according to the invention can be selected from OH, F, Cl, Br, I, CN, $NO_2$, I-$R^S_2$, NO, NCO, -NCS, -SCN, $SO_3H$, a monohalogenomethyl group, a dihalogenomethyl group, a trihalogenomethyl group, $CF(CF3)_2$, $SF_5$, aliphatic, aromatic, heteroarmatic, primary, secondary, tertiary amine or ammonium bound through N atom, - O-alkyl (alkoxy), -O-aryl, -O-heteroaryl -O-$SiR^S_3$,-S-S-$R^S$, -S-$R^S$, -S(O)-$R^S$, -$S(O)_2$-$R^S$, - COOH, -$CO_2$-$R^S$, -$BR^S_2$, -$PR^S_2$, -$OPR^S_2$, amide, bound through C or N atom, formyl group, - C(O)-$R^S$, -COOM, where M is a metal such as Li, Na, K, Cs, Ag. $R^S$ may be, independently from each other, the same or different and is each an aliphatic, heteroaliphatic, aromatic or heteroaromatic group, each optionally being further substituted by one or more heterosubstituents, aliphatic, heteroaliphatic, aromatic or heteroaromatic groups; and/or optionally bridged by an -O- atom, represents a halogenide

Aliphatic hydrocarbons including alkyl, alkenyl and alkinyl and may comprise straight-chain, branched and cyclic hydrocarbons.

Heteroaliphatic is a hydrocarbon including alkyl, alkenyl and alkinyl which may comprise straight-chain,

branched and cyclic hydrocarbons with one or more carbon atoms substituted with at least one heteroatom.

In more detail, $C_1$-$C_{20}$-alkyl can be straight chain or branched and has 1, 2, 3, 4, 5, 6, 7, 8, 9, 10, 11, 12, 13, 14, 15, 16, 17, 18, 19 or 20 carbon atoms. Alkyl might be $C_1$-$C_6$-alkyl, in particular methyl, ethyl, propyl, isopropyl, butyl, isobutyl, sec-butyl or tert-butyl, likewise pentyl, 1-, 2- or 3-methylpropyl, 1,1-, 1,2- or 2,2-dimethylpropyl, 1-ethylpropyl, hexyl, 1-, 2-, 3- or 4-methylpentyl, 1,1-, 1,2-, 1,3-, 2,2-, 2,3- or 3,3-dimethylbutyl, 1- or 2-ethylbutyl, 1-ethyl-1-methylpropyl, 1-ethyl-2-methylpropyl, 1,1,2- or 1,2,2-trimethylpropyl. Substituted alkyl groups are trifluoromethyl, pentafluoroethyl and 1,1,1-trifluoroethyl.

Cycloalkyl might be cyclopropyl, cyclobutyl, cyclopentyl, cyclohexyl or cycloheptyl. Alkenyl might be $C_2$-$C_{20}$ alkenyl. Alkinyl might be $C_2$-$C_{20}$ alkinyl.

Said unsaturated alkenyl- or alkinyl groups can be used for linking the inventive compounds to a carrier such as a polymer to serve for an immobilized catalyst.

Halogen is F, Cl, Br or I.

Alkoxy is preferably $C_1$-$C_{10}$ alkoxy such as methoxy, ethoxy, propoxy, tert-butoxy, butoxy, pentoxy, hexyloxy, etc and isomers thereof.

$C_3$-$C_8$-Heterocycloalkyl having one or more heteroatoms selected from among N, O and S is preferably 2,3-dihydro-2-, -3-, -4- or -5-furyl, 2,5-dihydro-2-, -3-, -4- or -5-furyl, tetrahydro-2- or -3-furyl, 1,3-dioxolan-4-yl, tetrahydro-2- or -3-thienyl, 2,3-dihydro-1-, -2-, -3-, -4- or -5-pyrrolyl, 2,5-dihydro-1-, -2-, -3-, -4- or -5-pyrrolyl, 1-, 2- or 3-pyrrolidinyl, tetrahydro-1-, -2- or-4-imidazolyl, 2,3-dihydro-1-, -2-, -3-, -4- or-5-pyrazolyl, tetrahydro-1-, -3- or-4-pyrazolyl, 1,4-dihydro-1-, -2-, -3- or -4-pyridyl, 1,2,3,4-tetrahydro-1-, -2-, -3-, -4-, -5- or -6-pyridyl, 1-, 2-, 3- or 4-piperidinyl, 2-, 3- or 4-morpholinyl, tetrahydro-2-, -3- or -4-pyranyl, 1,4-dioxanyl, 1,3-dioxan-2-, -4- or -5-yl, hexahydro-1-, -3- or -4-pyridazinyl, hexahydro-1-, -2-, -4- or -5-pyrimidinyl, 1-, 2- or 3-piperazinyl, 1,2,3,4-tetrahydro-1-, -2-, -3-, -4-, -5-, -6-, -7- or -8-quinolyl, 1,2,3,4-tetrahydro-1-, -2-, -3-, -4-, -5-, -6-, -7- or -8-isoquinolyl, 2-, 3-, 5-, 6-, 7- or 8-3,4-dihydro-2H-benzo-1,4-oxazinyl.

Optionally substituted means unsubstituted or monosubstituted, disubstituted, trisubstituted, tetrasubstituted, pentasubstituted, or even further substituted for each hydrogen, such as persubstituted, on the hydrocarbon.

Aryl might be a $C_6$ to $C_{22}$ aromatic hydrocarbon and may be phenyl, naphthyl, anthracenyl, phenanthryl or biphenyl

Arylalkyl might be benzyl.

Heteroaryl may be a $C_5$ to $C_{18}$ heteroaromatic hydrocarbon and may have one or more heteroatoms selected from among N, O and S, and is preferably 2- or 3-furyl, 2- or 3-thienyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, also preferably 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 3- or4-pyridazinyl, pyrazinyl, 1-, 2-, 3-, 4-, 5-, 6- or 7-Indolyl, 4- or 5-isoindolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 2-, 3-, 4-, 5-, 6-, 7- or 8-quinolyl, 1-, 3-, 4-, 5-, 6-, 7- or 8-isoquinolyl, 3-, 4-, 5-, 6-, 7- or 8-cinnolinyl, 2-, 4-, 5-, 6-, 7- or 8-quinazolinyl, 5- or 6-quinoxalinyl, 2-, 3-, 5-, 6-, 7-or 8-2H-benzo-1,4-oxazinyl, also preferably 1,3-benzodioxol-5-yl, 1,4-benzodioxan-6-yl, 2,1,3-benzothiadiazol-4- or -5-yl or 2,1,3-benzoxadiazol-5-yl.

The present invention is further illustrated by the attached Figures. In the Figures, it is shown in:

| | |
|---|---|
| Figure 1: | (A) Olefins as versatile building blocks for the synthesis of 1,2- and 1,3-difunctionalized compounds. (B) Pharmacologically active molecules containing 1,3-amino alcohol units. (C) The inventive approach: IDPi-catalyzed cycloaddition of unbiased olefins; |
| Scheme 1: Figure 2 a;b. | Yields and enantiomeric ratios for several reaction conditions and catalysts Substrate scope. Isolated yields after chromatographic purification. Enantiomeric ratios determined by HPLC or GC analysis. [a]Performed on 5 mmol scale. [b]Using 0.5 mol% catalyst loading [c]Reaction conducted in $Et_2O/CHCl_3$ (3:1 *v/v)* mixture at -30 °C. [d]Reaction conducted in $Et_2O/CHCl_3$ (3:1 *v/v*) mixture at -40 °C. [e]Reaction conducted in MTBE at -25 °C. [f]Reaction conducted in $Et_2O/CHCl_3$ (3:1 *v/v*) mixture at -10 °C. |
| Figure 3. | Synthesis of (R)-Fluoxetine hydrochloride from styrene. |

## Experimental Part

### Materials and Characterization

Unless otherwise stated, all reactions were magnetically stirred and conducted in oven-dried (90 °C) or flame-dried glassware in anhydrous solvents under argon, applying standard Schlenk techniques. Solvents and liquid reagents, as well as solutions of solid or liquid reagents were added via syringes, stainless steel or polyethylene cannulas through rubber septa or through a weak argon counter-flow. Solid reagents were added through a weak argon counter-flow. Cooling baths were prepared in Dewar vessels, filled with ice/water (0 °C), cooled acetone (< -78 °C) or dry ice/acetone (-78 °C). Heated oil baths were used for reactions requiring elevated temperatures. Solvents were removed under reduced pressure at 40 °C using a rotary evaporator, and unless otherwise stated, the remaining compound was dried in high vacuum ($10^{-3}$ mbar) at rt. All given yields are isolated yields of chromatographically and NMR-spectroscopically pure materials, unless otherwise stated.

Chemicals were purchased from commercial suppliers (including abcr, Acros, Alfa Aesar, Fluorochem, Sigma-Aldrich, and TCI) and used without further purification unless otherwise stated.

Solvents (CyH, DCM, $Et_2O$, THF, toluene) were dried by distillation from an appropriate drying agent in the technical department of the Max-Planck-Institut für Kohlenforschung and received in Schlenk flasks under argon. Other anhydrous solvents were purchased from commercial suppliers and used as received.

Reactions were monitored by thin layer chromatography (TLC) on silica gel pre-coated plastic sheets (0.2 mm, Macherey-Nagel). Visualization was accomplished by irradiation with UV light (254 nm and 366 nm) and/or phosphomolybdic acid (PMA) stain and/or Cerium Ammonium Molybdate (CAM) stain and/or permanganate stain.

Column chromatography was carried out using Merck silica gel (60 Å, 230-400 mesh, particle size 0.040-0.063 mm) using technical grade solvents. Elution was accelerated using compressed air. Automated column chromatography was conducted on a Biotage® IsoleraTM ISO-4SW instrument, using SNAP Ultra HP-SphereTM 25 μm chromatography cartridges. All fractions containing a desired substance were combined and concentrated in vacuo, then redissolved in an appropriate solvent and filtered through cotton to remove silica residues.

$^1H$, $^{13}C$, $^{19}F$, $^{31}P$ nuclear magnetic resonance (NMR) spectra were recorded on a Bruker AV-500, AV-400 or DPX-300 spectrometer in a suitable deuterated solvent. The solvent employed and respective measuring frequency are indicated for each experiment. Chemical shifts are reported with $Me_4Si$ serving as a universal reference of all nuclides and with two or one digits after the comma. The resonance multiplicity is described as s (singlet), d (doublet), t (triplet), q (quadruplet), p (pentet), hept (heptet), m (multiplet), and b (broad). All spectra were recorded at 298 K unless otherwise noted, processed with the program MestReNova 14.3, and coupling constants are reported as observed. The residual deuterated solvent signal relative to $Me_4Si$ was used as the internal reference in $^1H$ NMR spectra (e.g. $CDCl_3$ = 7.26 ppm) and are reported as follows: chemical shift in ppm (multiplicity, coupling constant J in Hz, number of protons). $^{13}C$, $^{19}F$, $^{31}P$ NMR spectra were referenced according to Ξ-values (IUPAC recommendations 2008)[2] relative to the internal references set in $^1H$ NMR spectra (e.g. $^{13}C$: $Me_4Si$, $^{19}F$: $CCl_3F$, $^{31}P$: $H_3PO_4$; each 0.00 ppm). All spectra are broadband decoupled unless otherwise noted.

Electron impact (EI) mass spectrometry (MS) was performed on a Finnigan MAT 8200 (70 eV) or MAT 8400 (70 eV) spectrometer. Electrospray ionization (ESI) mass spectrometry was conducted on a Bruker ESQ 3000 spectrometer. High resolution mass spectrometry (HRMS) was performed on a Finnigan MAT 95 (EI) or Bruker APEX III FTMS (7T magnet, ESI). The ionization method and mode of detection employed is indicated for the respective experiment and all masses are reported in atomic units per elementary charge (m/z) with an intensity normalized to the most intense peak.

Specific rotations $[a]_T^D$ were measured with a Rudolph RA Autopol IV Automatic Polarimeter at the indicated temperature (T) with a sodium lamp (sodium D line, λ = 589 nm). Measurements were performed in an acid resistant 1 mL cell (50 mm length) with concentrations (g/(100 mL)) reported in the corresponding solvent.

High-performance liquid chromatography (HPLC) was performed on Shimadzu LC-20AD liquid chromatograph (SIL-20AC auto sampler, CMB-20A communication bus module, DGU-20A5 degasser, CTO-20AC column oven, SPD-M20A diode array detector), Shimadzu LC-20AB liquid chromatograph (SIL-20ACHT auto sampler, DGU-20A5 degasser, CTO-20AC column oven, SPD-M20A diode array detector), or Shimadzu LC-20AB liquid chromatograph (reversed phase, SIL-20ACHT auto sampler, CTO-20AC column oven, SPD-M20A diode array detector) using Daicel columns with chiral stationary phases. All solvents used were HPLC-grade solvents, purchased from Merck. The column employed and respective solvent mixture are indicated for each experiment.

Liquid Chromatography-Mass Spectrometry Liquid chromatography-mass spectrometry (LC-MS) was performed on Shimadzu LC-MS 2020 liquid chromatograph. All solvents used were HPLC-grade solvents purchased from Sigma-Aldrich. The column employed, the respective solvent mixture, and the MS parameters are indicated for each experiment. Gas chromatography (GC) analyses on a chiral stationary phase were performed on HP 6890 and 5890 series instruments (split-mode capillary injection system, flame ionization detector (FID), hydrogen carrier gas). All of these analyses were conducted in the GC department of the Max-Planck-Institut für Kohlenforschung. The conditions employed are described in detail for the individual experiments.

**General Procedure A: IDPi-catalyzed Oxy-Aminomethylation of Olefins**

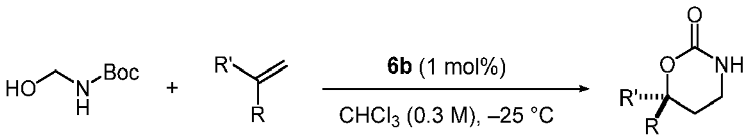


**1a** **2** **3**

An oven-dried screwcap vial (10 mL) equipped with a magnetic stir bar was charged with carbamate **1a** (0.5 mmol), olefin **2** (5 mmol, 10 equiv.) and dry $CHCl_3$ (1.5 mL). The tube was sealed and cooled down to the respective temperature for 30 min. After this time, a stock solution of catalyst **6b** in 0.1 mL of $CHCl_3$ (1 mol%) was added to the previous vial via syringe in one portion. After checking full conversion (TLC monitoring) the reaction was quenched with one equivalent of trimethylamine. The mixture was warmed up to room temperature, suspended on Celite, and further purified by silica gel column chromatography (DCM/MeOH mixtures from 0.5 to 3%).

**(R)-6-Phenyl-1,3-oxazinan-2-one (3a)**

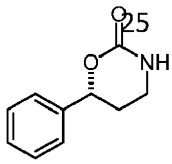

Following *General Procedure A,* employing **1a** (74 mg, 0.5 mmol, 1 equiv.) and olefin **2a** (0.57 mL, 5.0 mmol) as starting materials. After chromatographic purification, compound 3a was obtained as a white solid (62 mg, 70%). Spectroscopic data was consistent with the values reported in the literature.

**(*R*)-6-(naphthalen-2-yl)-1,3-oxazinan-2-one (3b)**

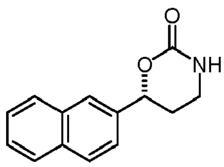

Following *General Procedure A,* employing **1a** (74 mg, 0.5 mmol, 1 equiv.) and olefin **2b** (770 mg, 5.0 mmol) as starting materials. After chromatographic purification, compound **3b** was obtained as a white solid (61 mg, 54%).

**(*R*)-6-(4-vinylphenyl)-1,3-oxazinan-2-one (3c)**

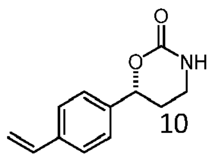

Following *General Procedure A,* employing **1a** (74 mg, 0.5 mmol, 1 equiv.) and olefin **2c** (0.65 g, 5.0 mmol) as starting materials. After chromatographic purification, compound 3c was obtained as a white solid (43 mg, 42%).

**(*R*)-6-(*p*-Tolyl)-1,3-oxazinan-2-one (3d)**

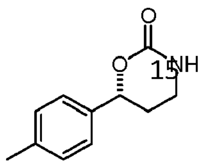

Following *General Procedure A,* employing **1a** (74 mg, 0.5 mmol, 1 equiv.) and olefin **2d** (0.66 mL, 5.0 mmol) as starting material, in $Et_2O/CHCl_3$ (3:1 *v*/*v)* mixture at -30 °C. After chromatographic purification, compound **3d** was obtained as a white solid (58 mg, 61%).

**(*R*)-6-(*o*-Tolyl)-1,3-oxazinan-2-one (3e)**

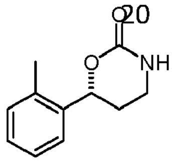

Following *General Procedure A,* employing **1a** (74 mg, 0.5 mmol, 1 equiv.) and olefin **2e** (0.65 mL, 5.0 mmol) as starting material. After chromatographic purification, compound 3e was obtained as a white solid (77 mg, 81%).

**(*R*)-6-(4-(*tert*-Butyl)phenyl)-1,3-oxazinan-2-one (3f)**

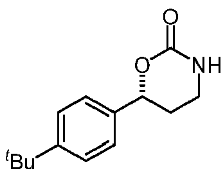

Following *General Procedure A,* employing **1a** (74 mg, 0.5 mmol, 1 equiv.) and olefin **2f** (0.91 mL, 5.0 mmol) as starting material, in $Et_2O/CHCl_3$ (3:1 *v*/*v*) mixture at -40 °C. After chromatographic purification, compound **3f** was obtained as a white solid (67 mg, 57%).

**(*R*)-6-(4-Fluorophenyl)-1,3-oxazinan-2-one (3g)**

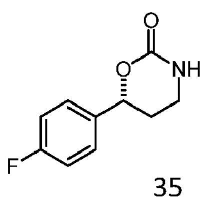

Following *General Procedure A,* employing **1a** (74 mg, 0.5 mmol, 1 equiv.) and olefin **2g** (0.60 mL, 5.0 mmol) as starting material. After chromatographic purification, compound **3g** was obtained as a white solid (69 mg, 71%).

**(*R*)-6-(4-Fluorophenyl)-1,3-oxazinan-2-one (3h)**

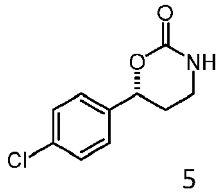

Following *General Procedure A,* employing **1a** (74 mg, 0.5 mmol, 1 equiv.) and olefin **2h** (0.63 mL, 5.0 mmol) as starting material. After chromatographic purification, compound **3h** was obtained as a white solid (58 mg, 55%).

**(*R*)-6-(4-Bromophenyl)-1,3-oxazinan-2-one (3i)**

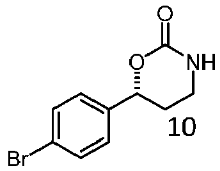

Following *General Procedure A,* employing **1a** (74 mg, 0.5 mmol, 1 equiv.) and olefin **2i** (0.63 mL, 5.0 mmol) as starting material. After chromatographic purification, compound **3i** was obtained as a white solid (58 mg, 55%).

**(*R*)-4-(2-Oxo-1,3-oxazinan-6-yl)phenyl acetate (3j)**

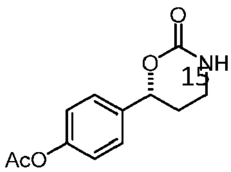

Following *General Procedure A,* employing **1a** (74 mg, 0.5 mmol, 1 equiv.) and olefin **2j** (0.77 mL, 5.0 mmol) as starting material, in $Et_2O/CHCl_3$ (3:1 *v/v)* mixture at -30 °C. After chromatographic purification, compound **3j** was obtained as a white solid (66 mg, 56%).

**(*R*)-6-(4-(Chloromethyl)phenyl)-1,3-oxazinan-2-one (3k)**

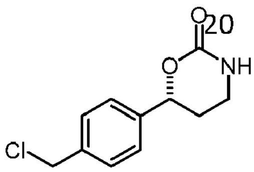

Following *General Procedure A,* employing **1a** (74 mg, 0.5 mmol, 1 equiv.) and olefin **2k** (0.70 mL, 5.0 mmol) as starting material. After chromatographic purification, compound **3k** was obtained as a white solid (57 mg, 51%).

**(R)-6-(3-Methoxyphenyl)-1,3-oxazinan-2-one (3l)**

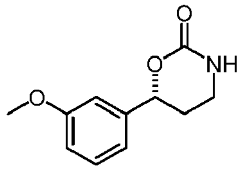

Following *General Procedure A,* employing **1a** (74 mg, 0.5 mmol, 1 equiv.) and olefin **2l** (0.69 mL, 5.0 mmol) as starting material. After chromatographic purification, compound **3l** was obtained as a white solid (68 mg, 67%).

**(*R*)-6-(3-Bromophenyl)-1,3-oxazinan-2-one (3m)**

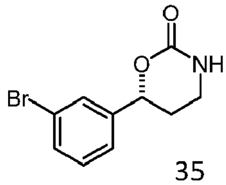

Following *General Procedure A,* employing **1a** (74 mg, 0.5 mmol, 1 equiv.) and olefin **2m** (0.65 mL, 5.0 mmol) as starting material. After chromatographic purification, compound **3m** was obtained as a white solid (55 mg, 43%).

**(*R*)-6-(3-Bromo-4-methylphenyl)-1,3-oxazinan-2-one (3n)**

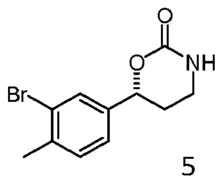

Following *General Procedure A,* employing **1a** (74 mg, 0.5 mmol, 1 equiv.) and olefin **2n** (0.65 mL, 5.0 mmol) as starting material. After chromatographic purification, compound **3n** was obtained as a white solid (68 mg, 71%).

**(*R*)-6-(Thiophen-3-yl)-1,3-oxazinan-2-one (3o)**

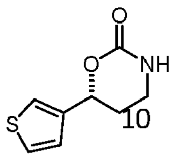

Following *General Procedure A,* employing **1a** (74 mg, 0.5 mmol, 1 equiv.) and olefin **2o** (0.37 mL, 5.0 mmol) as starting material, in MTBE at -30 °C. After chromatographic purification, compound **3o** was obtained as a white solid (71 mg, 78%).

**(*R*)-6-(Benzo[b]thiophen-6-yl)-1,3-oxazinan-2-one (3p)**

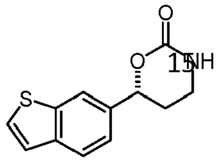

Following *General Procedure A,* employing **1a** (44 mg, 0.3 mmol, 1 equiv.) and olefin **2p** (480 mg, 3.0 mmol) as starting material, in $Et_2O/CHCl_3$ (3:1 *v/v)* mixture at -30 °C. After chromatographic purification, compound **3p** was obtained as a white solid (53 mg, 76%).

**HPLC** (Chiralpak IC-3, n-heptane/i-PrOH 50:50, 298 K, 220 nm): $t_R$ (major) = 17.1 min, $t_R$ (minor) = 22.4 min, e.r. = 95.5:4.5 (91% e.e.).

**(*R*)-6-(Benzofuran-6-yl)-1,3-oxazinan-2-one (3q)**

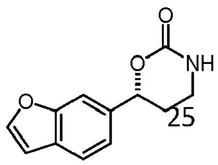

Following *General Procedure A,* employing **1a** (37 mg, 0.25 mmol, 1 equiv.) and olefin **2q** (360 mg, 2.5 mmol) as starting material. After chromatographic purification, compound **3q** was obtained as a white solid (47 mg, 87%).

**(*R*)-6-Ethyl-6-phenyl-1,3-oxazinan-2-one (3r)**

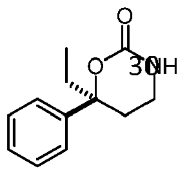

Following *General Procedure A,* employing **1a** (57 mg, 0.4 mmol, 1 equiv.) and olefin **2s** (528 mg, 4.0 mmol) as starting material, in $Et_2O/CHCl_3$ (3:1 *v/v)* mixture at -30 °C. After chromatographic purification, compound **3s** was obtained as a white solid (59 mg, 71%).

**(*R*)-6-Cyclopentyl-6-phenyl-1,3-oxazinan-2-one (3s)**

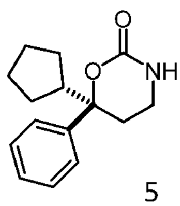

Following *General Procedure A,* employing **1a** (34 mg, 0.25 mmol, 1 equiv.) and olefin **2t** (400 mg, 2.5 mmol) as starting material, in $Et_2O/CHCl_3$ (3:1 *v/v)* mixture at -30 °C. After chromatographic purification, compound **3t** was obtained as a white solid (13 mg, 23%).

**(*R*)-6-isopropyl-6-phenyl-1,3-oxazinan-2-one (3t)**

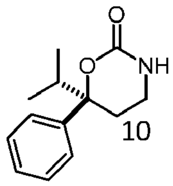

Following *General Procedure A,* employing **1a** (57 mg, 0.4 mmol, 1 equiv.) and olefin **2u** (528 mg, 4.0 mmol) as starting material. After chromatographic purification, compound **3u** was obtained as a white solid (38 mg, 43%).

**(*R*)-6-Cyclohexyl-6-phenyl-1,3-oxazinan-2-one (3u)**

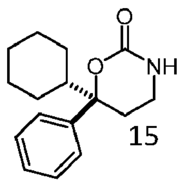

Following *General Procedure A,* employing **1a** (59 mg, 0.4 mmol, 1 equiv.) and olefin **2v** (745 mg, 4.0 mmol) as starting material. After chromatographic purification, compound **3v** was obtained as a white solid (58 mg, 56%).

**(*S*)-6-isopropyl-6-methyl-1,3-oxazinan-2-one (3v)**

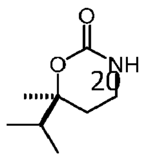

Following *General Procedure A,* employing **1a** (74 mg, 0.5 mmol, 1 equiv.) and olefin **2w** (0.62 mL, 5.0 mmol) as starting material in MTBE. After chromatographic purification, compound **3w** was obtained as a white solid (47 mg, 60%).

**(*S*)-6-(*tert*-Butyl)-6-methyl-1,3-oxazinan-2-one (3w)**

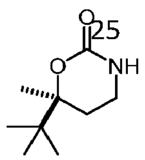

Following *General Procedure A,* employing **1a** (74 mg, 0.5 mmol, 1 equiv.) and olefin **2w** (0.70 mL, 5.0 mmol) as starting material, in $Et_2O/CHCl_3$ (3:1 *v*/*v*) mixture at -10 °C. After chromatographic purification, compound **3w** was obtained as a white solid (30 mg, 35%).

**Scale-Up Experiments**

### 1.1. Catalyst and Olefin Recovery Experiment

An oven-dried Schleck flask equipped with a magnetic stir bar was charged with carbamate **1a** (735 mg, 5.0 mmol, 1 equiv.), olefin **2a** (3.7 mL, 50 mmol) and dry $CHCl_3$ (12 mL). The flask was sealed and cooled down to -25 °C for 30 min. After this time, a stock solution of catalyst **6b** in 4 mL of $CHCl_3$ (42 mg, 0.5 mol%) was added dropwise via syringe. After the reaction was completed (TLC monitoring) it was quenched with one equivalent of trimethylamine and stirred for 30 min at rt. The reaction crude mixture was transferred to a round-bottomed flask to facilitate manipulation. A short distillation apparatus was attached to the flask and $CHCl_3$ and trimethylamine were distilled off the crude under vacuum (100 mbar, rt). The receiving flask was changed and the non-reacted styrene was further recovered by bulb-to-bulb vacuum distillation (94% styrene recovered, 1 mbar, 35 °C). The crude residue was purified by silica gel column chromatography (DCM/MeOH mixtures from 0.5 to 3%) to give 545 mg of 3a as a white solid (62%, e.r. = 96.5:3.5). Fractions containing IDPi catalyst were combined and purified by silica gel column chromatography (n-hexane/EtOAc 9:1 to 4:1) to afford a white solid, which was subjected to acidification by filtration over a plug of DOWEX 50WX8 (H-form, eluted with DCM) to obtain the reisolated catalyst **6b** (30 mg, 72%) as a yellowish solid.

### 1.2. Formal Synthesis of (R)-Fluoxetine Hydrochloride from Styrene 2a

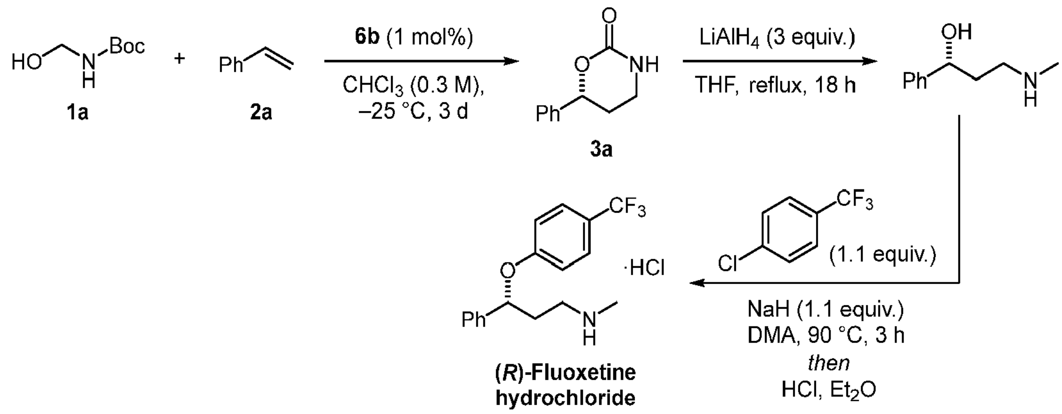


**(*R*)-6-Phenyl-1,3-oxazinan-2-one (3a)**

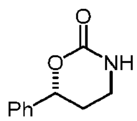

*Following General Procedure A:* an oven-dried Schleck flask equipped with a magnetic stir bar was charged with carbamate **1a** (2.5 g, 17 mmol, 1 equiv.), olefin **2a** (19.5 mL, 170 mmol) and dry $CHCl_3$ (40 mL). The flask was sealed and cooled down to -25 °C for 30 min. After this time, a stock solution of catalyst **6b** in 14 mL of $CHCl_3$ (283 mg, 1 mol%) was added dropwise via syringe. After the reaction was completed (TLC monitoring) it was quenched with one equivalent of trimethylamine and stirred for 30 min at rt. The mixture was suspended on Celite and further purified by silica gel column chromatography (DCM/MeOH mixtures from 0.5 to 3%) to give 2.20 g **3a** as a white solid (73%, e.r. = 96.5:3.5).

**(*R*)-3-(Methylamino)-1-phenylpropan-1-ol**

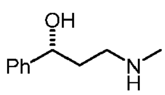

Following a reported procedure: to a two-necked round-bottomed oven-dried Schleck flask charged with product **3a** (2.20

g, 73 mmol, 1 equiv.) and dry THF (124 mL) was added $LiAlH_4$ (1.4 mg, 37 mmol, 3 equiv.) under argon at 0 °C. The mixture was refluxed overnight. After cooling down to rt, the reaction was diluted with MTBE (30 mL), and cooled down to 0 °C. $LiAlH_4$ is quenched by a careful addition of water (3 mL), followed by an aqueous NaOH 15% solution (3 mL), and water (15 mL). The mixture is warmed up to rt and stirred for 30 min. Then it was transferred to a separatory funnel and both phases were separated. The aqueous phase was extracted with MTBE (3x10 mL). Combined organic phases were washed with brine (1x10 mL), dried over anhydrous $Na_2SO_4$ and evaporated to give (R)-3-(methylamino)-1-phenylpropan-1-ol as a pure pale yellow oil (1.86 g, 91%) without further purification. Spectroscopic data was consistent with the values reported in the literature.

**(R)-Fluoxetine hydrochloride**

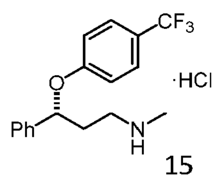

15

Following a reported procedure: in a flame-dried Schlenk under argon atmosphere, (R)-3-(methylamino)-1-phenylpropan-1-ol (1.60 g, 9.7 mmol, 1 equiv.) was dissolved in 9 mL of dry dimethylacetamide. The mixture was cooled to 0 °C and sodium hydride (0.46 g, 11.6, 1.2 equiv., 60% dispersion in paraffin liquid) was added slowly. The mixture was heated at 90 °C for 1.5 h, and an orange solution resulted. To this solution was added 4-chlorobenzotrifluoride (1.92 g, 10.7 mmol, 1.1 equiv.), and the mixture was heated at 105°C for 2 h. After cooling to rt and dilution with EtOAc (10 mL), the mixture was washed with water (5 mL), and the aqueous layer was separated and extracted with EtOAc (3x5 mL). The combined organic solutions were washed with sat. aq. NaHCOs (1x10 mL) and brine (1x10 mL), dried over anhydrous $Na_2SO_4$ and evaporated. The crude free base Fluoxetine was dissolved in 10 mL $Et_2O$ and acidified with gaseous HCl to afford 3.05 g (91% yield) of (R)-Fluoxetine hydrochloride as pale yellow crystals. Spectroscopic data was consistent with the values reported in the literature.

As noted above, chiral 1,3-amino alcohols are ubiquitous structural motives in natural products and active pharmaceutical ingredients, and the inventors here present the enantioselective strong Brønsted acid-catalyzed [4+2] cycloaddition of unbiased olefins and iminium ions, which provides a one-step approach for the direct transformation of simple alkenes into highly functionalized molecules under mild reaction conditions.

## Claims

1. Process for the asymmetric cycloaddition of an iminium ion precursor to an alkene as represented in the following reaction scheme:

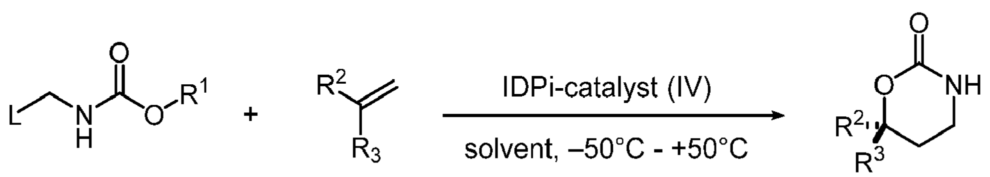


(I) (II) (III)

wherein an iminium ion precursor (I) is reacted with an alkene of Formula (II) in the presence of a catalyst of the Formula (IV), preferably in the presence of an organic solvent, whereby a 1,3-oxazinan-2-one of Formula (III) is obtained,
wherein:

$R^1$ represents H, $C_1$-$C_{18}$-alkyl, including tert-butyl, 3-ethylpentan-3-yl, 3-methylpentan-3-yl, tert-pentyl, $C_6$-$C_{14}$ aryl or $C_1$ -$C_6$ alkyl substituted $C_6$-$C_{14}$ aryl including benzyl, 2-phenylpropan-2-yl, 1,1-diphenylethyl,
L stands for a leaving group selected from hydroxy, alkoxy, acetate, sulfonate and halogen,
$R^2$ represents H, $C_1$-$C_6$-alkyl, $C_1$-$C_6$-cycloalkyl, $C_1$-$C_8$-alkenyl, $C_1$-$C_8$-alkynyl, $C_6$ to $C_{18}$ aromatic hydro-

carbon or $C_5$ to $C_{18}$ heteroaromatic hydrocarbon, each aromatic or heteroaromatic hydrocarbon optionally being substituted by one or more substituents selected from halogen, $C_1$ to $C_{20}$ straight chain, branched chain or cyclic aliphatic hydrocarbons, $C_2$ to $C_6$ alkenyl, $C_6$ to $C_{18}$ aromatic hydrocarbons or $C_5$ to $C_{18}$ heteroaromatic hydrocarbons, optionally having one or more halogens, preferably F and/or Cl, $SF_5$, $NO_2$ or cyano on the aliphatic, aromatic or heteroaromatic hydrocarbon,
$R^3$ represents hydrogen, $C_1$-$C_6$-alkyl, or -$C_1$-$C_6$-cycloalkyl, each alkyl optionally being substituted by halogen, and
wherein the IDPi-catalyst is represented by Formula (IV)

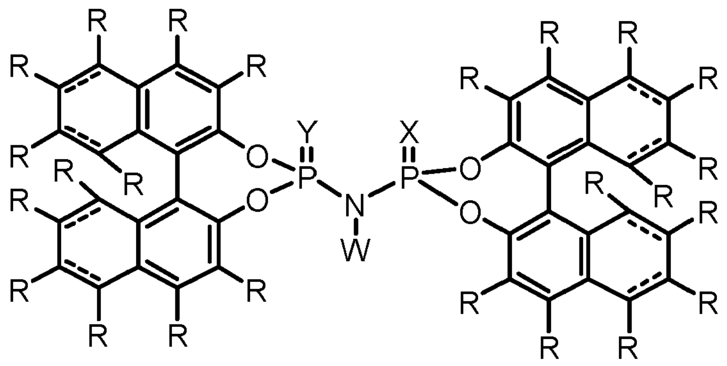
(IV)

wherein in said formula (IV):

R is the same or different on each position and is each selected from hydrogen, halogen, $SF_5$, $NO_2$, cyano, $C_1$ to $C_{20}$ straight chain, branched chain or cyclic aliphatic hydrocarbons, optionally having one or more halogens, preferably F or C!, $SF_5$, $NO_2$ or cyano on the aliphatic hydrocarbon, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain, branched chain or cyclic aliphatic hydrocarbons, $C_6$ to $C_{18}$ aromatic hydrocarbons or $C_5$ to $C_{18}$ heteroaromatic hydrocarbons optionally having one or more halogens, preferably F and/or C!, $SF_5$, $NO_2$ or cyano on the aliphatic or aromatic hydrocarbon,
wherein any dashed line independently represents a double bond or two hydrogens,
wherein X and Y are the same or different and represent $NR^N$; wherein:
$R^N$ is an electron withdrawing group, being the same or different on each position and being selected from:

i. -alkyl, -CO-alkyl, -(CO)-O-alkyl, sulfinyl alkyl, sulfonyl alkyl, sulfonyl iminoalkyl, sulfonyl bisiminoalkyl, phosphinyl dialkyl, phosphonyl alkyl, alkyl phosphorane, N,N'-alkylimidazolidin-2-iminyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or Cl, cyano, nitro, or $SF_5$;
ii. -aryl, -CO-aryl, -(CO)-O-aryl, sulfinyl aryl, sulfonyl aryl, sulfonyl iminoaryl, sulfonyl iminosulfonylaryl, sulfonyl bisiminoaryl, phosphinyl diaryl, phosphinyl alkylaryl, phosphonyl aryl, aryl phosphoranes, aryl alkyl phosphoranes, N,N'-arylimidazolidin-2-iminyl, N-aryl-N'-alkylimidazolidin-2-iminyl, wherein aryl is a $C_6$ to $C_{18}$ aromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;
iii. -heteroaryl, -CO-heteroaryl, -(CO)-O-heteroaryl, sulfinyl heteroaryl, sulfonyl heteroaryl, -(P=O)-di-heteroaryl, phosphinyl diheteroaryl, phosphinyl arylheteroaryl, phosphinyl heteroaryl alkyl, phosphonyl heteroaryl, heteroaryl phosphoranes, heteroaryl aryl phosphoranes, heteroaryl aryl alkyl phosphoranes, N,N'-heteroarylimidazolidin-2-iminyl, N-heteroaryl-N'-alkylimidazolidin-2-iminyl, N-heteroaryl-N'-arylimidazolidin-2-iminyl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or Cl, cyano, nitro, or $SF_5$;
and

wherein W is selected from hydrogen, halogen, a metal selected from Li, Na, K, Rb, Cs, Be, Mg, Ca, Sr, Ba Sc, Ti, V, Cr, Mn, Fe, Co, Ni, Cu, Zn, Y, Zr, Mo, Ru, Rh, Pd, Ag, Cd, W, Re, Os, Ir, Pt, Au, Hg, Al, Ga, In, Ge, Sn, Pb, As, Sb, Bi, Se, Te, La, Sm, Eu, Yb, U or a cationic organic group, a substituted borane $-BR^IR^{II}R^{III}$ or a substituted silicon $-SiR^IR^{II}R^{III}$, wherein $R^1$, $R^{II}$ and $R^{III}$ may be same or different and each stands for hydrogen, halogen, an optionally -O- bonded $C_1$ to $C_{20}$ straight chain, branched chain or cyclic aliphatic hydrocarbon, optionally having one or more unsaturated bonds or one or more hetero atoms in the chain, a $C_5$ to $C_{18}$ heteroaromatic hydrocarbon, a $C_6$ to $C_{18}$ aromatic hydrocarbon or partially arene-hydrogenated forms thereof, each hydrocarbon optionally being substituted by one or more groups selected from $C_1$ to $C_{20}$ straight chain, branched chain or cyclic aliphatic hydrocarbons, or one or more heterosubstituents, W being preferably selected from hydrogen, alkali metal or earth alkaline metal.

**2.** Process according to claim 1, wherein in Formula (I), (II) and (III),

PG, L and the catalyst of Formula (IV) are as defined in claim 1,
$R^1$ represents H, $C_1$-$C_6$-alkyl, or -$C_1$-$C_6$-cycloalkyl, $C_6$ to $C_{18}$ aromatic hydrocarbon or $C_5$ to $C_{18}$ heteroaromatic hydrocarbon, each aromatic or heteroaromatic hydrocarbon optionally being substituted by one or more substituents selected from halogen, $C_1$ to $C_{20}$ straight chain, branched chain or cyclic aliphatic hydrocarbons, $C_2$ to $C_6$ alkenyl, $C_6$ to $C_{18}$ aromatic hydrocarbons or $C_5$ to $C_{18}$ heteroaromatic hydrocarbons optionally having one or more halogens, preferably F and/or C!, $SF_5$, $NO_2$ or cyano on the aliphatic or aromatic hydrocarbon,
$R^2$ represents hydrogen, $C_1$-$C_6$-alkyl, or -$C_1$-$C_6$-cycloalkyl, each alkyl optionally being substituted by halogen.

**3.** Process according to claim 1 or 2, wherein the catalyst is represented by the following formula (IVa) or (IVb) :

(IVa)

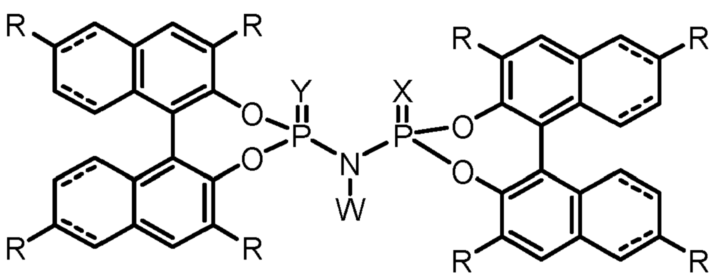

(IVb)

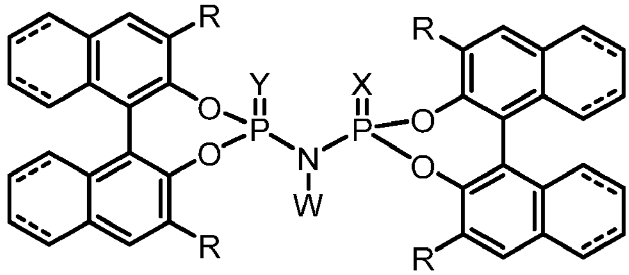

wherein the substituent R is the same or different on each position and is defined as in claim 1,
X and Y have the meanings as defined in claim 1,
any dashed line independently represents two hydrogens or preferably a double bond and W represents hydrogen, an alkali metal or an earth alkaline metal.

**4.** Process according to any one of claims 1 to 3, wherein the substituent R is the same or different on each position and represents halogen, a straight chain, branched chain or cyclic $C_1$ to $C_{20}$ aliphatic hydrocarbon or a $C_6$ to $C_{18}$ aromatic hydrocarbon, said aliphatic hydrocarbon and/or aromatic hydrocarbon being substituted by one or more halogens, preferably F and/or Cl, $SF_5$, $NO_2$ or a straight chain, branched chain or cyclic $C_1$ to $C_{20}$ aliphatic hydrocarbon, optionally being substituted by one or more halogens, preferably F and/or Cl, $SF_5$, $NO_2$ on the aliphatic hydrocarbon.

**5.** Process according to any one of the preceding claims, wherein, in any formula (IV), (IVa) or (IVb), X and Y are the same or different and represent $NR^N$, wherein $R^N$ is an electron withdrawing group, being the same or different on each

position and being selected from:

j. sulfinyl alkyl or sulfonyl alkyl, wherein alkyl is a $C_1$ to $C_{20}$ straight chain, branched chain or cyclic aliphatic hydrocarbon, optionally having at least one substituent selected from $C_1$ to $C_6$ alkoxy, halogen, preferably F and/or C!, cyano, nitro or $SF_5$;
ii. sulfinyl aryl, or sulfonyl aryl, wherein aryl is a $C_6$ to $C_{18}$ aromatic hydrocarbon, optionally having at least one substituent selected from $C_1$ to $C_6$ alkyl optionally substituted by at least one halogen , $C_1$ to $C_6$ alkoxy, halogen, preferably F and/or C!, cyano, nitro or $SF_5$;
iii. sulfinyl heteroaryl, or sulfonyl heteroaryl, wherein heteroaryl is a $C_2$ to $C_{18}$ heteroaromatic hydrocarbon, optionally having at least one substituent selected from $C_1$ to $C_6$ alkyl optionally substituted by at least one halogen, $C_1$ to $C_6$ alkoxy, halogen, preferably F and/or C!, cyano, nitro or $SF_5$; and

wherein R and W have the meanings as defined in any one of the preceding claims.

**6.** Process according to any one of the preceding claims, wherein the catalyst is represented by Formula (IVc)

(IVc)

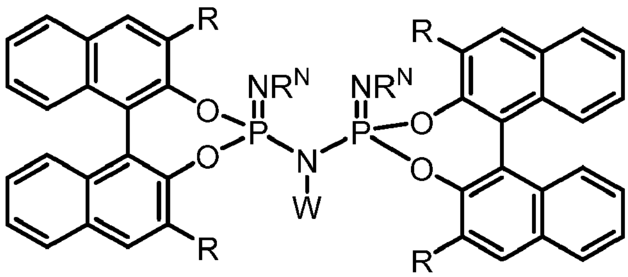

wherein R is the same on each position and is selected from hydrogen, $C_6$ to $C_{18}$ aromatic hydrocarbons or $C_5$ to $C_{18}$ heteroaromatic hydrocarbons, each aromatic or heteroaromatic hydrocarbon optionally being substituted by one or more substituents selected from halogen, $SF_5$, $NO_2$, cyano, $C_1$ to $C_{20}$ straight chain,
branched chain or cyclic aliphatic hydrocarbons, $C_6$ to $C_{18}$ aromatic hydrocarbons or $C_5$ to $C_{18}$ heteroaromatic hydrocarbons, optionally having one or more halogens, preferably F and/or Cl, $SF_5$, $NO_2$ or cyano on the aliphatic, aromatic or heteroaromatic hydrocarbon.

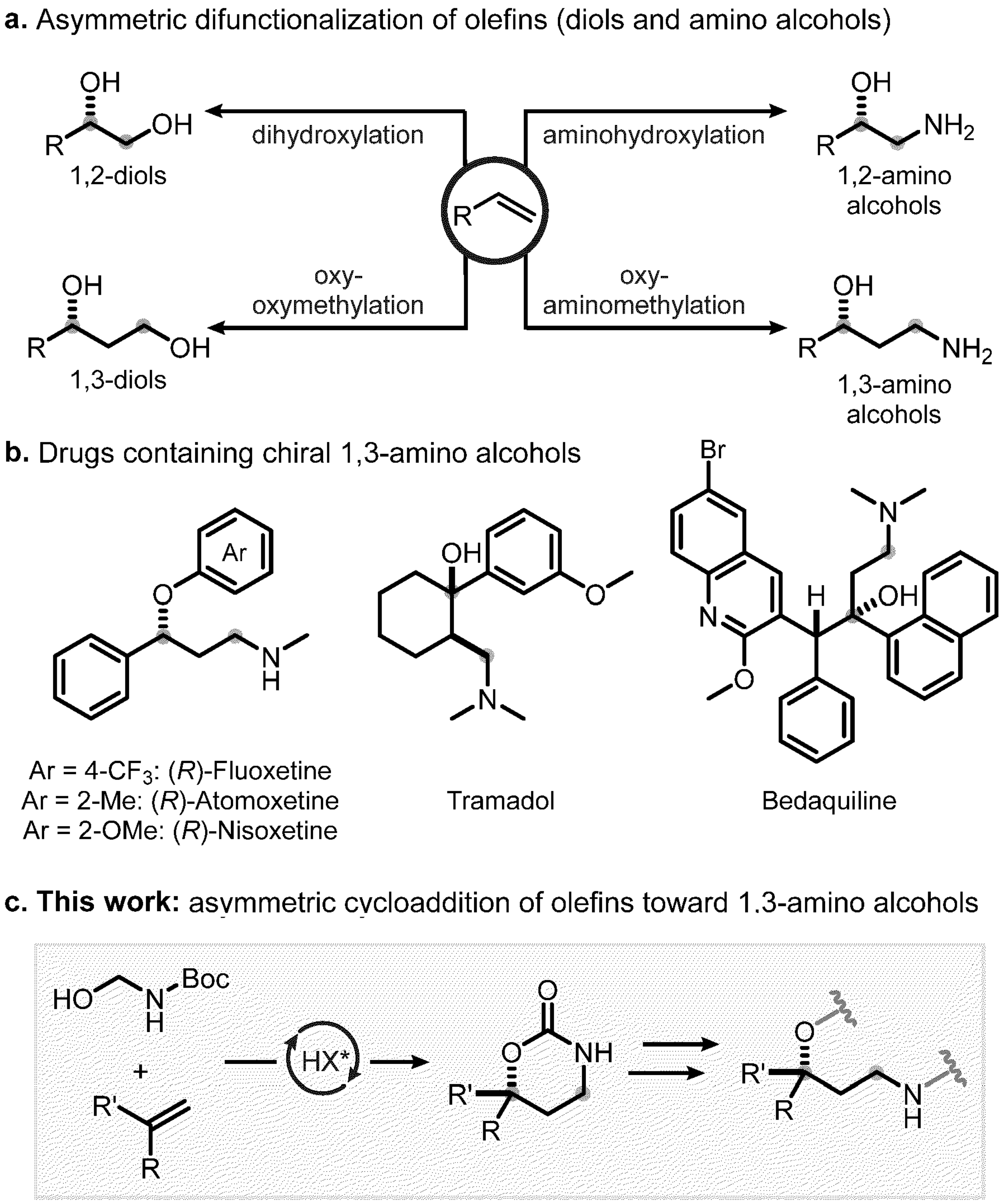
a. Asymmetric difunctionalization of olefins (diols and amino alcohols)
dihydroxylation
aminohydroxylation
1,2-diols
1,2-amino alcohols
oxy-oxymethylation
oxy-aminomethylation
1,3-diols
1,3-amino alcohols
b. Drugs containing chiral 1,3-amino alcohols
Ar = 4-$CF_3$: (*R*)-Fluoxetine
Ar = 2-Me: (*R*)-Atomoxetine
Ar = 2-OMe: (*R*)-Nisoxetine
Tramadol
Bedaquiline
c. This work: asymmetric cycloaddition of olefins toward 1,3-amino alcohols

Figure 1

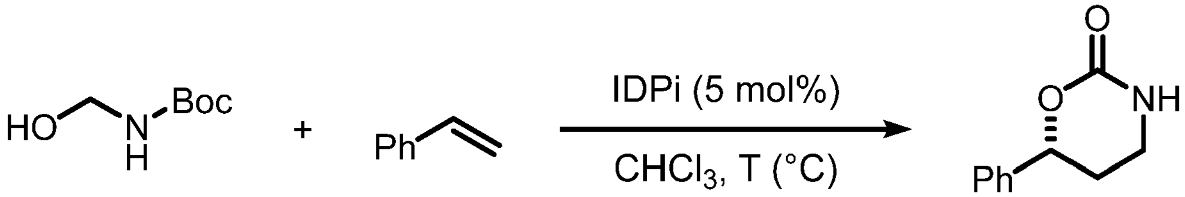


**1a** + **2a** (equiv.) → **3a**

| entry[a] | IDPi | T (°C) | 2a equiv. | yield (%) | e.r. |
|---|---|---|---|---|---|
| 1 | 4a | rt | 20 | 56 | 68:32 |
| 2 | 5a | rt | 20 | 64 | 72:18 |
| 3 | 6a | rt | 20 | 56 | 82:18 |
| 4 | 6b | rt | 20 | 50 | 93:7 |
| 5 | 6b | –25 | 10 | 75 | 97:3 |
| **6[b]** | **6b** | **–25** | **10** | **73** | **97:3** |
| 7[b] | 7b | –25 | 10 | 61 | 93:7 |
| 8[b] | 6b | –25 | 5 | 51 | 97:3 |
| 9[c,d] | 6b | –25 | 10 | 62 | 96.5:3.5 |

**IDPi**

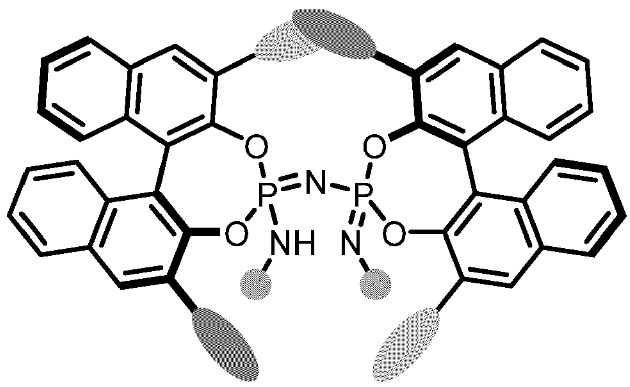

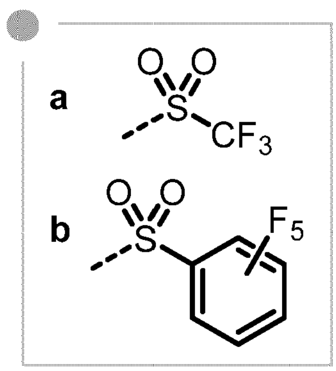

**a**

**b**

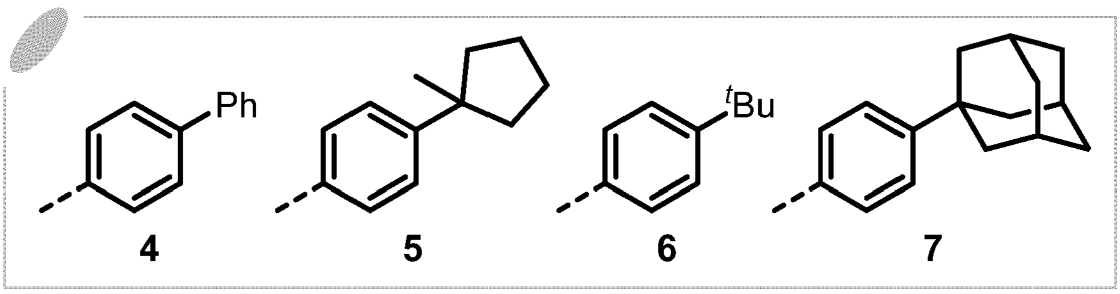

**4** **5** **6** **7**

Scheme 1

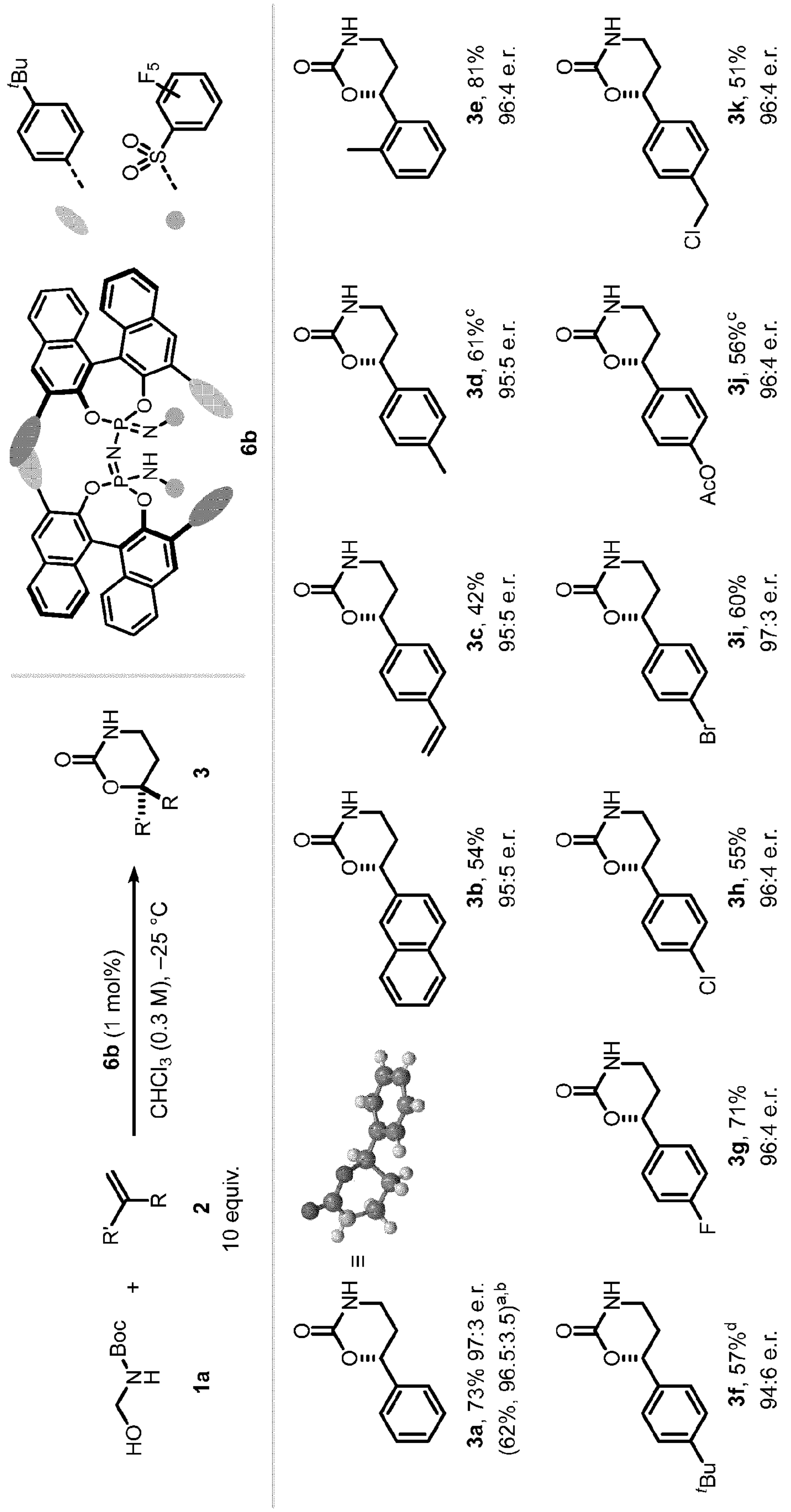
1a
2
10 equiv.
6b (1 mol%)
$CHCl_3$ (0.3 M), −25 °C
3
6b
3a, 73% 97:3 e.r.
(62%, 96.5:3.5)[a,b]
3b, 54%
95:5 e.r.
3c, 42%
95:5 e.r.
3d, 61%[c]
95:5 e.r.
3e, 81%
96:4 e.r.
3f, 57%[d]
94:6 e.r.
3g, 71%
96:4 e.r.
3h, 55%
96:4 e.r.
3i, 60%
97:3 e.r.
3j, 56%[c]
96:4 e.r.
3k, 51%
96:4 e.r.

Figure 2a

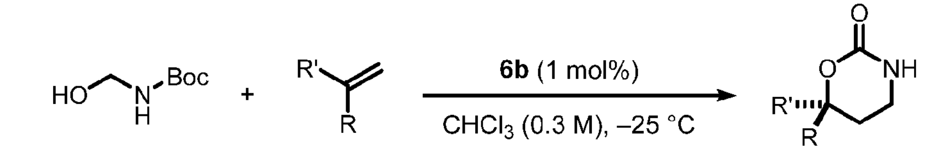


1a + 2 (10 equiv.) → 3

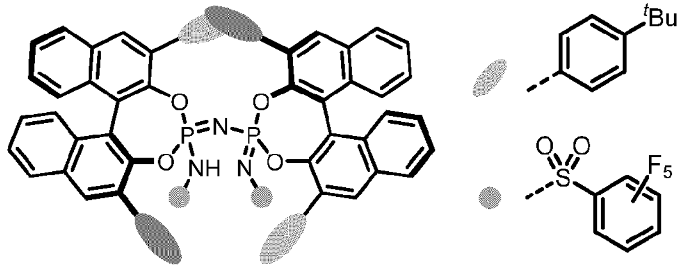

6b

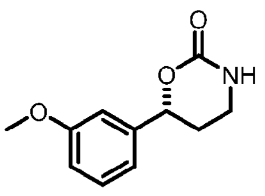

**3l**, 67%
96:4 e.r.

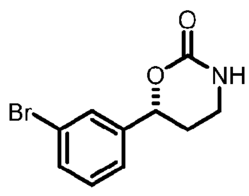

**3m**, 43%
97:3 e.r.

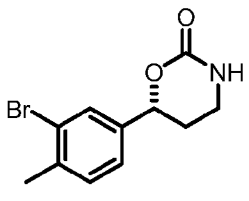

**3n**, 71%
97:3 e.r.

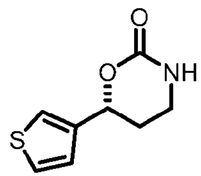

**3o**, 78%[e]
97:3 e.r.

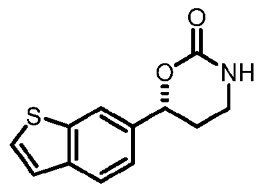

**3p**, 76%[c]
95.5:4.5 e.r.

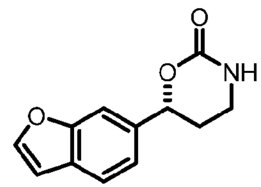

**3q**, 61%[d]
93:7 e.r.

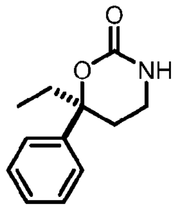

**3r**, 71%[c]
95:5 e.r.

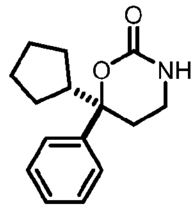

**3s**, 23%[c]
95:5 **e.r.**

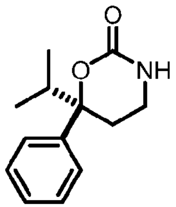

**3t**, 43%
97.5:2.5 e.r.

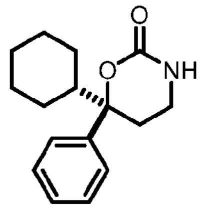

**3u**, 56%[c]
97:3 e.r.

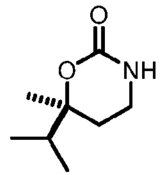

**3v**, 78%[f]
93:7 e.r.

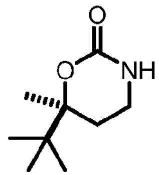

**3w**, 20%[f]
95:5 e.r.

Figure 2b

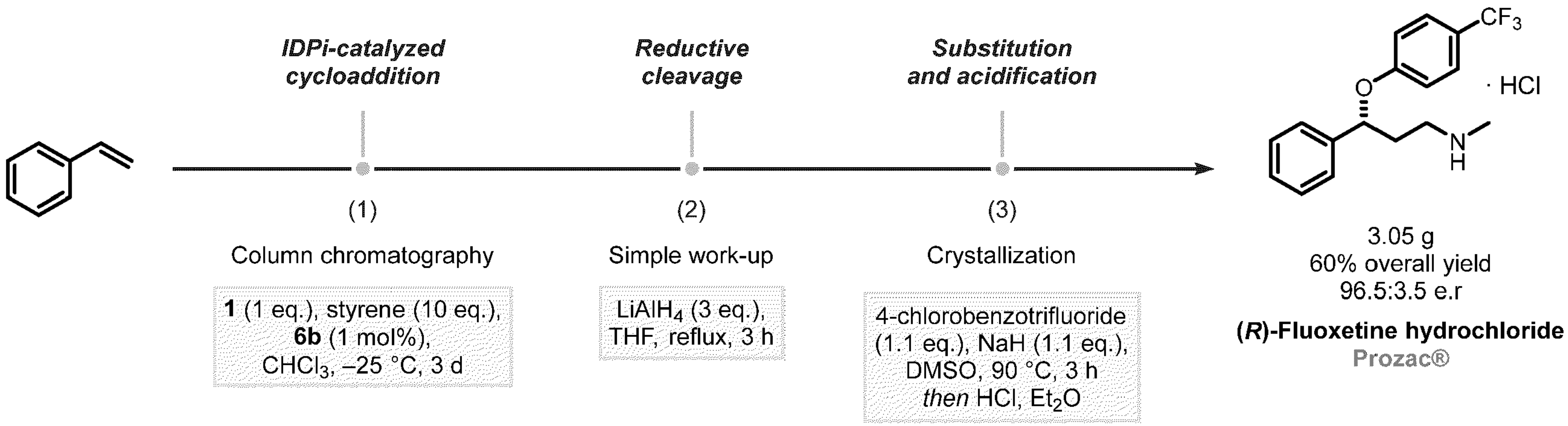
IDPi-catalyzed cycloaddition
(1)
Column chromatography
1 (1 eq.), styrene (10 eq.), 6b (1 mol%), CHCl3, –25 °C, 3 d
Reductive cleavage
(2)
Simple work-up
LiAlH4 (3 eq.), THF, reflux, 3 h
Substitution and acidification
(3)
Crystallization
4-chlorobenzotrifluoride (1.1 eq.), NaH (1.1 eq.), DMSO, 90 °C, 3 h then HCl, Et2O
· HCl
3.05 g
60% overall yield
96.5:3.5 e.r
(R)-Fluoxetine hydrochloride
Prozac®

Figure 3

Europäisches Patentamt
European Patent Office
Office européen des brevets

# EUROPEAN SEARCH REPORT

Application Number
EP 24 18 4448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CZOMBIK ANNA M ET AL: "Magnesium-catalyzed stereoselective transformations - A survey through recent achievements", POLYHEDRON, PERGAMON PRESS, OXFORD, GB, vol. 219, 23 March 2022 (2022-03-23), XP087027357, ISSN: 0277-5387, DOI: 10.1016/J.POLY.2022.115790 [retrieved on 2022-03-23] * abstract * * scheme 18 *; page 18 ----- | 1-6 | INV. C07D265/10 |
| A | CN 105 017 172 A (CHINESE ACAD INST CHEMISTRY) 4 November 2015 (2015-11-04) * abstract * * examples 1-13 * * claims 1-9 * ----- | 1-6 | |
| A | NIMMAGADDA SRI KRISHNA ET AL: "Asymmetric One-Pot Synthesis of 1,3-Oxazolidines and 1,3-Oxazinanes via Hemiaminal Intermediates", ORGANIC LETTERS, vol. 16, no. 16, 30 July 2014 (2014-07-30), pages 4098-4101, XP093186158, US ISSN: 1523-7060, DOI: 10.1021/ol501789c * abstract * * page 4100; figure 3 * ----- -/-- | 1-6 | TECHNICAL FIELDS SEARCHED (IPC) C07D |

The present search report has been drawn up for all claims

1

| Place of search | Date of completion of the search | Examiner |
|---|---|---|
| Munich | 4 December 2024 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 5

Europäisches Patentamt
European Patent Office
Office européen des brevets

# EUROPEAN SEARCH REPORT

Application Number
EP 24 18 4448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VINCENT BARBIER ET AL: "[beta]-Lactams as Formal Dipoles through Amide-Bond Activation", EUROPEAN JOURNAL OF ORGANIC CHEMISTRY, WILEY-VCH, DE, vol. 2016, no. 3, 9 December 2015 (2015-12-09), pages 549-555, XP072110154, ISSN: 1434-193X, DOI: 10.1002/EJOC.201501342 * abstract * * scheme 2 *; page 549 * page 550; table 2 * * page 551; table 3 * ----- | 1-6 | |
| A | GHORAI ET AL: "Lewis acid mediated SN2-type nucleophilic ring opening followed by [4+2] cycloaddition of N-tosylazetidines with aldehydes and ketones: synthesis of chiral 1,3-oxazinanes and 1,3-amino alcohols", TETRAHEDRON LETTERS, ELSEVIER, AMSTERDAM , NL, vol. 48, no. 25, 23 May 2007 (2007-05-23), pages 4373-4377, XP022093142, ISSN: 0040-4039, DOI: 10.1016/J.TETLET.2007.04.097 * abstract * * schemes 1, 2 *; page 4374; table 1 ----- -/-- | 1-6 | TECHNICAL FIELDS SEARCHED (IPC) |

The present search report has been drawn up for all claims

1

| Place of search | Date of completion of the search | Examiner |
|---|---|---|
| Munich | 4 December 2024 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 18 4448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NAZIM UDDIN ET AL: "Bronsted acid-catalyzed C-C bond forming reaction for one step synthesis of oxazinanones", TETRAHEDRON LETTERS, ELSEVIER, AMSTERDAM , NL, vol. 52, no. 33, 13 June 2011 (2011-06-13) , pages 4353-4356, XP028237766, ISSN: 0040-4039, DOI: 10.1016/J.TETLET.2011.06.054 [retrieved on 2011-06-18] * abstract * * scheme 1 *; page 4353 * page 4354; tables 1-3 * * schemes 2-4 *; page 4355; table 4 ----- | 1-6 | |
| A | JAKOB UWE ET AL: "Intramolecular conversion of N,N-bis(2-picolyl)ureas to cyclic carbamates", TETRAHEDRON LETTERS, vol. 56, no. 46, 28 September 2015 (2015-09-28), pages 6340-6344, XP029321099, ISSN: 0040-4039, DOI: 10.1016/J.TETLET.2015.09.118 * abstract * * scheme 1 *; page 6341 * page 6342 - page 6343; table 2 * ----- -/-- | 1-6 | TECHNICAL FIELDS SEARCHED (IPC) |

The present search report has been drawn up for all claims

1

| Place of search | Date of completion of the search | Examiner |
|---|---|---|
| Munich | 4 December 2024 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 18 4448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BORAH ARUN JYOTI ET AL: "Synthesis of new 4,6-disubstituted-1,3-oxazinan-2-one analogues", JOURNAL OF CHEMICAL SCIENCES, SPRINGER INDIA, NEW DELHI, vol. 125, no. 6, 24 December 2013 (2013-12-24), pages 1503-1510, XP035310107, ISSN: 0974-3626, DOI: 10.1007/S12039-013-0508-5 [retrieved on 2013-12-24] * abstract * * scheme 1 *; page 1507 * page 1508; table 1 * ----- | 1-6 | |
| A | FADY NAHRA ET AL: "Striking AcOH Acceleration in Direct Intramolecular Allylic Amination Reactions", CHEMISTRY - A EUROPEAN JOURNAL, JOHN WILEY & SONS, INC, DE, vol. 15, no. 42, 30 September 2009 (2009-09-30), pages 11078-11082, XP071829800, ISSN: 0947-6539, DOI: 10.1002/CHEM.200901946 * abstract * * page 11078; table 1 * * page 11079; table 2 * ----- | 1-6 | TECHNICAL FIELDS SEARCHED (IPC) |
| | -/-- | | |

The present search report has been drawn up for all claims

1

| Place of search | Date of completion of the search | Examiner |
|---|---|---|
| Munich | 4 December 2024 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 18 4448

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CONGMIN MEI ET AL: "Synthesis of Oxazolidinones and Derivatives through Three-Component Fixation of Carbon Dioxide", CHEMCATCHEM, JOHN WILEY & SONS, INC, HOBOKEN, USA, vol. 10, no. 14, 28 May 2018 (2018-05-28), pages 3057-3068, XP072439773, ISSN: 1867-3880, DOI: 10.1002/CCTC.201800142 * abstract * * scheme 1 *; page 3057 * page 3060; table 3 * ----- | 1-6 | |
| A | DAS BUBUL ET AL: "Synthetic utility of styrenes in the construction of diverse heterocycles via annulation/cycloaddition", TETRAHEDRON, ELSEVIER SIENCE PUBLISHERS, AMSTERDAM, NL, vol. 134, 20 January 2023 (2023-01-20), XP087282125, ISSN: 0040-4020, DOI: 10.1016/J.TET.2023.133270 [retrieved on 2023-01-20] * abstract * * scheme 29 *; page 9 * scheme 64 *; page 18 ----- | 1-6 | |
| | | | TECHNICAL FIELDS SEARCHED (IPC) |

The present search report has been drawn up for all claims

1

| Place of search | Date of completion of the search | Examiner |
|---|---|---|
| Munich | 4 December 2024 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 18 4448

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2024

| Patent document cited in search report | Publication date | Patent family member(s) | Publication date |
|---|---|---|---|
| CN 105017172 A | 04-11-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 23198382
- EP 20200632


### Non-patent literature cited in the description


- **HOSSAIN**. *Tetrahedron Letters*, 2011, vol. 52 (33), 4353-4356
- **LIST et al.** *J. Am. Chem. Soc.*, 2016, vol. 138 (34), 10822